(19) Europäisches Patentamt
European Patent Office
Office européen des brevets

(11) Publication number: 0 200 403
B1

(12) **EUROPEAN PATENT SPECIFICATION**

(45) Date of publication of the patent specification: 25.01.89

(51) Int. Cl.[4]: C 07 C 43/225, C 07 C 43/12, C 07 C 41/22

(21) Application number: 86302694.4

(22) Date of filling: 11.04.86

(54) **Preparation of alkoxy halides.**

(30) Priority: 16.04.85 GB 8509730

(43) Date of publication of application: 05.11.86 Bulletin 86/45

(45) Publication of the grant of the patent: 25.01.89 Bulletin 89/4

(84) Designated Contracting States: BE CH DE FR GB IT LI NL

(56) References cited:
EP-A-0 185 554
CH-A-385 386

CHEMICAL ABSTRACTS, vol. 64, no. 13, 20th June 1966, abstract no. 19549d, Columbus, Ohio, US; Y.I. CHUMAKOV et al.: "Cetylpyridinium chloride", & METODY POLUCHENIYA KHIM. REAKTIVOV. I. PREPARATOV. 11, 105-7 (1964)
REAGENTS FOR ORGANIC SYNTHESIS, vol. 6, 1974, page 220, John Wiley & Sons, New York, US; "Dimethylformamide-phosphorus pentachloride"
Methoden der organischen Chemie (Houben-Weyl) VI/1b, pp. 931-932 (1984)
Römpps Chemie Lexikon, Ed. 7, Vol. 6, 3812 (1977)

(73) Proprietor: The British Petroleum Company p.l.c., Britannic House Moor Lane, London EC2Y 9BU (GB)

(72) Inventor: Hodgson, Philip Kenneth Gordon, The British Petroleum p.l.c. Chertsey Road, Sunbury-on- Thames Middlesex, TW16 7LN (GB)
Inventor: Stewart, Nevin John, The British Petroleum p.l.c. Chertsey Road, Sunbury- on-Thames Middlesex, TW16 7LN (GB)

(74) Representative: MacLeod, Malcolm, BP INTERNATIONAL LIMITED Patents Division Chertsey Road, Sunbury- on- Thames Middlesex TW16 7LN (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

EP 0 200 403 B1

LIBER, STOCKHOLM 1989

## Description

This invention relates to a process for the preparation of alkyl alkoxy, aryl alkoxy or alkylaryl alkoxy halides suitable for use as intermediates in the preparation of surfactants.

Numerous attempts have been made to develop surfactant compositions for use in enhanced oil recover and the patent literature is replete with descriptions of formulations, see for example, USP's 4 424 135, 4 159 037, 4 110 228, 4 066 124 and 4 018 278.

A useful summary of the art is given in Kirk-Othmer's Encyclopedia of Chemical Technology, Third Edition, Volume 17, pages 168 - 182. This indicates that most compositions contain

(a) a main surfactant which is either a petroleum or a synthetic hydrocarbyl sulphonate and

(b) co-surfactants which include simple alcohols, ethoxylated alcohols and sulphated ethoxylated alcohols.

It has also been disclosed that alkyl and alkylaryl polyalkoxy alkylene sulphonates may be used as co-surfactants. These compounds are generally prepared by a three-stage process. In the first stage of a typical process an alcohol or alkyl phenol is condensed with an alkylene oxide in the presence of sodium or potassium hydroxide to form an alkoxylate. This is then halogenated by treatment with thionyl or sulphuryl chloride, usually in the absence of a catalyst. Finally the halide is converted to a sulphonate by reaction with sodium sulphite, again, usually in the absence of a catalyst.

We have now discovered that the use of an amide catalyst in the second stage, the halogenation stage, results in a shorter reaction period and an increased yield of desired product.

Thus according to the present invention there is provided a process for the production of an alkyl, aryl or alkylaryl alkoxy halide which process comprises reacting an alkyl-, aryl- or alkylaryl-alkoxy alcohol of formula:

$R - (OR^1)_m OH$

wherein

R is an alkyl group, containing 1 to 24, preferably 8 to 20, carbon atoms, a phenyl group or an alkyl aryl group of formula:

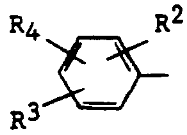

wherein $R^2$ is an alkyl group containing 1 to 24, preferably 8 to 20, carbon atoms and

$R^3$ and $R^4$ are hydrogen atoms, or

$R^2$ and $R^3$ are both alkyl groups containing 1 to 12 carbon atoms and

$R^4$ is a hydrogen atom, or

$R^2$, $R^3$ and $R^4$ are each alkyl groups wherein the sum of the number of carbon atoms in the groups is in the range 3 to 24;

$R^1$ is an alkylene group containing 2 or 3 carbon atoms; and m is a number in the range 1 to 15 preferably 4 to 10, with a halogenating agent in the presence of an aliphatic amide or urea as catalyst.

Suitable catalysts include the hydrochlorides of the aliphatic amides, and ureas. Suitable aliphatic amides include N,N-dimethylformamide and acetamide.

Preferred halogenating agents are chlorinating agents such as thionyl chloride and sulphuryl chloride.

The reaction is suitably effected at a temperature in the range 30° to 120° C, preferably in the range 75° to 85° C.

Pressure is not a significant parameter and the reaction is therefore most conveniently carried out at ambient pressure.

The molar ratio of alkoxy alcohol to halogenating agent is suitably in the range 1 : 1 to 1 : 5, preferably 1 : 1 to 1 : 1.5.

The quantity of catalyst employed is preferably in the range 0.1 to 5 % by weight, expressed as a percentage by weight of the alkoxy alcohol.

The reaction may be effected in the presence or, preferably, the absence of a solvent. Suitable solvents include 1,2-dichloroethane, toluene and chloroform.

As a result of the reduced reaction time, the tendency of the polyoxyalkylene chain to cleave liberating 1,4-dioxan, noted by Robinson et al, J. Soc. Cosmet. Chem., 31, 329 - 337, is reduced. Thus both the yield and selectivity as indicated by the matching of the alkoxy distribution in the alcohol and the halide are improved and product contamination with unwanted by-product is reduced.

The invention is illustrated with reference to the following Examples. Example 1, hovever, is not in accordance with the invention and is provided for comparison.

### Example 1

61.1 g (0.11M) of the ethoxy alcohol $C_{18}H_{37}Ph(OCH_2CH_2)_5OH$ was heated with stirring at 80° C with 13.9 g (0.12M) thionyl chloride in the absence of catalyst and solvent.

$^{13}C$ NMR (Nuclear Magnetic Resonance) spectroscopy indicated quantitative conversion to ethoxychloride after 3 hours and GLC (Gas Liquid Chromatography) indicated the production of 0.07 mole of 1,4-dioxan per mole ethoxy alcohol.

**Example 2**

64.6 g (0.11M) of the ethoxy alcohol $C_{18}H_{37}Ph(OCH_2CH_2)_5OH$ was heated with stirring at 80°C with 14.7 g (0.12M) thionyl chloride and 0.65 g (1 %) N,N-dimethylformamide in the absence of solvent.

$^{13}C$ NMR spectroscopy indicated quantitative chlorination after 0.25 hours and GLC indicated the production of 0.008 mole of 1,4-dioxan per mole ethoxy alcohol.

**Example 3**

67.0 g (0.12M) of the ethoxy alcohol $C_{18}H_{37}Ph(OCH_2CH_2)_5OH$ was heated with stirring at 80°C with 15.2 g (0.13M) thionyl chloride and 0.67 g (1 %) urea in the absence of solvent.

$^{13}C$ NMR spectroscopy indicated quantitative chlorination after 1 hour and GLC indicated the production of 0.01 mole of 1,4-dioxan per mole ethoxy alcohol.

**Example 4**

62.1 g (0.11M) of the ethoxyalcohol $C_{18}H_{37}Ph(OCH_2CH_2)_5OH$ was heated with stirring at 80°C with 14.2 g (0.12M) thionyl chloride and 0.62 g (1 %) acetamide in the absence of solvent.

$^{13}C$ NMR spectroscopy indicated quantitative chlorination after 2 hours and GLC indicated the production of 0.03 mole of 1,4-dioxan per mole ethoxyalcohol.

When compared with Example 1, Examples 2, 3 and 4 show a substantial decrease in dioxan production when a catalyst in accordance with the invention is used, together with a considerable increase in the chlorination rate.

**Claims**

1. A process for the production of an alkyl-, aryl- or alkylaryl alkoxy halide which process comprises reacting an alkyl, aryl, or alkylaryl alkoxy alcohol of formula:

$R\text{-}(OR^1)_mOH$

wherein R is an alkyl group containing 1 to 24 carbon atoms, a phenyl group, or an alkylaryl group of formula:

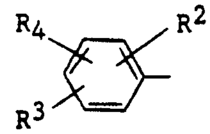

wherein
$R^2$ is an alkyl group containing 1 to 24 carbon atoms and
$R^3$ and $R^4$ are hydrogen atoms, or
$R^2$ and $R^3$ are both alkyl groups containing 1 to 12 carbon atoms and
$R^4$ is a hydrogen atom, or
$R^2$, $R^3$ and $R^4$ are each alkyl groups wherein the sum of the number of carbon atoms in the groups is in the range 3 to 24;
$R^1$ is an alkylene group containing 2 or 3 carbon atoms; and
m is a number in the range 1 to 15, with a halogenating agent,
characterised by the fact that the reaction is carried out in the presence of an aliphatic amide or urea as catalyst.

2. A process according to claim 1 wherein R is an alkylaryl group of formula:

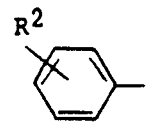

wherein
$R^2$ is an alkyl group containing 8 to 20 carbon atoms and
m is a number in the range 4 to 10.

3. A process according to claim 1 werein the aliphatic amide is N,N-dimethylformamide or acetamide.

4. A process according to any of the preceding claims wherein the halogenating agent is thionyl chloride or sulphuryl chloride.

5. A process according to any of the preceding claims wherein the halogenation reaction is effected at a temperature in the range 30 to 120°C.

6. A process according to any of the preceding claims wherein the molar ratio of alkoxy alcohol to halogenating agent is in the range 1 : 1 to 1 : 5.

7. A process according to any of the preceding claims wherein the quantity of catalyst employed is in the range 0.1 % to 5 % by weight, expressed as a percentage by weight of the alkoxy alcohol.

**Patentansprüche**

1. Verfahren zur Herstellung eines Alkyl-, Aryl- oder Alkylarylalkoxyhalogenids, bei dem ein Alkyl-, Aryl- oder Alkylarylalkoxyalkohol der Formel:

$R-(OR^1)_mOH$

in der

R eine Alkyl-Gruppe mit 1 bis 24 Kohlenstoff-Atomen, eine Phenyl-Gruppe oder eine Alkylaryl-Gruppe der Formel

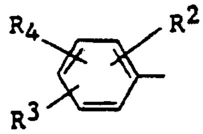

ist, in der

$R^2$ eine Alkyl-Gruppe mit 1 bis 24 Kohlenstoff-Atomen ist und

$R^3$ und $R^4$ Wasserstoff-Atome sind oder

$R^2$ und $R^3$ beide Alkyl-Gruppen mit 1 bis 12 Kohlenstoff-Atomen sind und

$R^4$ ein Wasserstoff-Atom ist oder

$R^2$, $R^3$ und $R^4$ jeweils Alkyl-Gruppen sind, wobei die Summe der Zahl der Kohlenstoff-Atome in den Gruppen im Bereich von 3 bis 24 liegt,

$R^1$ eine Alkylen-Gruppe mit 2 oder 3 Kohlenstoff-Atomen ist und

m eine Zahl im Bereich von 1 bis 15 ist, mit einem Halogenierungsmittel umgesetzt wird, dadurch gekennzeichnet, daß die Reaktion in Gegenwart eines aliphatischen Amids oder von Harnstoff als Katalysator durchgeführt wird.

2. Verfahren nach Anspruch 1, worin R eine Alkylaryl-Gruppe der Formel:

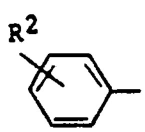

ist, in der

$R^2$ eine Alkyl-Gruppe mit 8 bis 20 Kohlenstoff-Atomen ist und

m eine Zahl im Bereich von 4 bis 10 ist.

3. Verfahren nach Anspruch 1, worin das aliphatische Amid N,N-Dimethylformamid oder Acetamid ist.

4. Verfahren nach irgendeinem der vorhergehenden Ansprüche, worin das Halogenierungsmittel Thionylchlorid oder Sulfurylchlorid ist.

5. Verfahren nach irgendeinem der vorhergehenden Ansprüche, worin die Halogenierungs-Reaktion bei einer Temperatur im Bereich von 30°C bis 120° durchgeführt wird.

6. Verfahren nach irgendeinem der vorhergehenden Ansprüche, worin das Stoffmengen-Verhältnis ("Molverhältnis") des Alkoxyalkohols zu dem Halogenierungsmittel im Bereich von 1 : 1 bis 1 : 5 liegt.

7. Verfahren nach irgendeinem der vorhergehenden Ansprüche, worin die eingesetzte Menge des Katalysators im Bereich von 0,1 bis 5 Gew.-%, ausgedrückt als Prozentsatz des Gewichts des Alkoxyalkohols, liegt.

**Revendications**

1. Procédé pour la production d'un halogénure d'alcoxy alkyle, d'alcoxy aryle ou d'alcoxy alkylaryle, ce procédé comprenant la réaction d'un alkyl alcoxy alcool, d'un aryl alcoxy alcool ou d'un alkylaryl alcoxy alcool de formule:

$R-(OR^1)_mOH$

dans laquelle:

R représente un groupe alkyle contenant 1 à 24 atomes de carbone, un groupe phényle ou un groupe alkylaryle de formule:

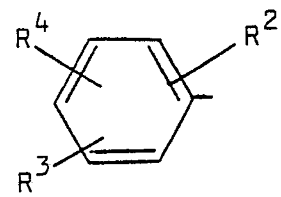

dans laquelle:

$R^2$ représente un groupe alkyle contenant 1 à 24 atomes de carbone et

$R^3$ et $R^4$ représentent chacun un atome d'hydrogène, ou bien

$R^2$ et $R^3$ représentent chacun un groupe alkyle contenant 1 à 12 atomes de carbone et

$R^4$ représente un atome d'hydrogène, ou bien

$R^2$, $R^3$ et $R^4$ représentent chacun un groupe alkyle, la somme du nombre des atomes de carbone présents dans les groupes se situant entre 3 et 24;

$R^1$ représente un groupe alkylène contenant 2 ou 3 atomes de carbone; et

<u>m</u> est un nombre valant 1 à 15,

avec un agent d'halogénation,

procédé caractérisé par le fait que l'on effectue la réaction en présence d'un amide aliphatique ou d'une urée comme catalyseur.

2. Procédé selon la revendication 1, dans lequel R représente un groupe alkylaryle de formule:

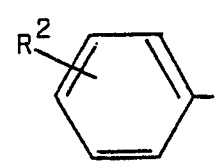

dans laquelle:

$R^2$ représente un groupe alkyle contenant 8 à 20 atomes de carbone, et

m est un nombre valant 4 à 10.

3. Procédé selon la revendication 1, dans lequel l'amide aliphatique est le N,N-diméthylformamide ou l'acétamide.

4. Procédé selon l'une quelconque des revendications précédentes, dans lequel l'agent d'halogénation est le chlorure de thionyle ou le chlorure de sulfuryle.

5. Procédé selon l'une quelconque des revendications précédentes, dans lequel on effectue la réaction d'halogénation en opérant à une température comprise entre 30° et 120°.

6. Procédé selon l'une quelconque des revendications précédentes, dans lequel le rapport molaire de l'alcoxy alcool à l'agent d'halogénation se situe entre 1 : 1 et 1 : 5.

7. Procédé selon l'une quelconque des revendications précédentes, dans lequel la quantité de catalyseur que l'on utilise se situe entre 0,1 % et 5 % en poids, la quantité étant exprimée en pourcentage pondéral de l'alcoxy alcool.